(19) Europäisches Patentamt
European Patent Office
Office européen des brevets

(11) Publication number: **0 194 464 B1**

(12) # EUROPEAN PATENT SPECIFICATION

(45) Date of publication of patent specification: **03.04.91**

(51) Int. Cl.[5]: **C07C 237/12**, C07C 323/52, A61K 31/16, A61K 37/02

(21) Application number: **86101865.3**

(22) Date of filing: **14.02.86**

(54) **Amino acid derivatives and use thereof for the preparation of an anticonvulsant.**

(30) Priority: **15.02.85 US 702195**

(43) Date of publication of application:
**17.09.86 Bulletin 86/38**

(45) Publication of the grant of the patent:
**03.04.91 Bulletin 91/14**

(84) Designated Contracting States:
**AT BE CH DE FR GB IT LI LU NL SE**

(56) References cited:
**EP-A- 0 007 441**
**EP-A- 0 046 707**
**GB-A- 1 051 220**
**US-A- 3 707 559**
**EP-A- 0 038 758**
**DE-A- 1 927 692**
**US-A- 2 676 188**

**TETRAHEDRON, vol. 33, no. 5, 1977, pages 489-495, Pergamon Press, GB; M. IKEDA et al.: "Photochemical synthesis of 1,2,3,4-tetrahydroisoquinolin-3-ones from N-chloroacetylbenzylamines"**

(73) Proprietor: **Research Corporation Technologies, Inc. (a Delaware corp.)**
**6840 East Broadway Boulevard**
**Tucson Arizona 85710-2815(US)**

(72) Inventor: **Kohn, Harold L.**
**3735 Latma Drive**
**Houston, TX(US)**
Inventor: **Watson, Darrell**
**801 North Pearl**
**Belton, TX(US)**

(74) Representative: **Eitle, Werner, Dipl.-Ing. et al**
**Hoffmann, Eitle & Partner Patentanwälte Arabellastrasse 4**
**W-8000 München 81(DE)**

**EP 0 194 464 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

TETRAHEDRON LETTERS, vol. 25, no. 42, 1984, pages 4841-4844, Pergamon Press Ltd., GB; J. GARCIA et al.: "New synthetic "tricks". Triphenylphosphine-mediated amide formation from carboxylic acids and azides"

JOURNAL OF THE AMERICAN CHEMICAL SOCIETY, vol. 106, no. 2, 25th January 1984, pages 457-459, American Chemical Society, US; B.H. LIPSHUTZ et al.: "Heterocycles in synthesis: Chiral amino acids/dipeptides via a novel photooxidative cleavage of trisubstituted imidazoles"

JOURNAL OF THE AMERICAN CHEMICAL SOCIETY, vol. 105, no. 26, 28th December 1983, pages 7703-7713, American Chemical Society, US; B.H. LIPSHUTZ et al.: "Heterocycles as masked diamide/dipeptide equivalents. Formation and reactions of substituted 5-(acylamino)oxazoles as intermediates en route to the cyclopeptide alkaloids"

JOURNAL OF MEDICINAL CHEMISTRY, vol. 28, no. 5, 1985, pages 601-606; S. CORTES et al.: "Effect of structural modification of the hydaution ring on anticonvulsant activity"

## Description

The predominant application of anticonvulsant drugs is the control and prevention of seizures associated with epilepsy or related central nervous system disorders. Epilepsy refers to many types of recurrent seizures produced by paroxysmal excessive neuronal discharges in the brain; the two main generalized seizures are petit mal, which is associated with myoclonic jerks, akinetic seizures, transient loss of consciousness, but without convulsion; and grand mal which manifests itself in a continuous series of seizures and convulsions with loss of consciousness.

The mainstay of treatment for such disorders has been the long-term and consistent administration of anticonvulsant drugs. Most drugs in use are weak acids that, presumably, exert their action on neurons, glial cells or both of the central nervous system. The majority of these compounds are characterized by the presence of at least one amide unit and one or more benzene rings that are present as a phenyl group or part of a cyclic system.

Much attention has been focused upon the development of anticonvulsant drugs and today many such drugs are well known. For example, the hydantoins, such as phenytoin, are useful in the control of generalized seizures and all forms of partial seizures. The oxazolidinediones, such as trimethadione and paramethadione, are used in the treatment of nonconvulsive seizures. Phenacemide, a phenylacetylurea, is one of the most well known anticonvulsants employed today, although much attention has recently been dedicated to the investigation of the diazepines and piperazines. For example, U.S. Patent Nos. 4,002,764 and 4,178,378 to Allgeier, et al. disclose esterified diazepine derivatives useful in the treatment of epilepsy and other nervous disorders. U.S. Patent No. 3,887,543 to Nakanishi, et al. describes a thieno [2,3-e][1,4] diazepine compound also having anticonvulsant activity and other depressant activity. U.S. Patent No. 4,209,516 to Heckendorn, et al. relates to triazole derivatives which exhibit anticonvulsant activity and are useful in the treatment of epilepsy and conditions of tension and agitation. Finally, U.S. Patent No. 4,372,974 to Fish, et al. discloses a pharmaceutical formulation containing an aliphatic amino acid compound in which the carboxylic acid and primary amino are separated by three or four units. Administration of these compounds in an acid pH range are useful in the treatment of convulsion disorders and also possess anxiolytic and sedative properties.

Unfortunately, despite the many available pharmacotherapeutic agents, a significant percentage of the population with epilepsy or related disorders are poorly managed. Moreover, none of the drugs presently available are capable of achieving total seizure control and most have disturbing side-effects. Clearly, current therapy has failed to "seize control" of these debilitating diseases.

DE 1 927 692 discloses amino acid derivatives, the closest of which are N-phenylacetyl-D,L-alanine-N-methylamide and N-phenylacetyl-D,L-phenylglycine-N-methylamide. Both of these prior art compounds demonstrated no anticonvulsant activity under standard testing procedures at dosage levels above 300 mg $kg^{-1}$ while the closest compounds of the present application, N-acetyl-D,L-alanine-N-benzylamide and N-acetyl-D,L-phenylglycine-N-benzylamide, exhibit activity at levels less than 100 mg $kg^{-1}$.

It is one object of the present invention to provide novel compounds exhibiting anticonvulsant activity.

Another object of this invention is to provide pharmaceutical compositions useful in the treatment of epilepsy and other central nervous system disorders.

The present invention is directed to a compound having the general formula (I):

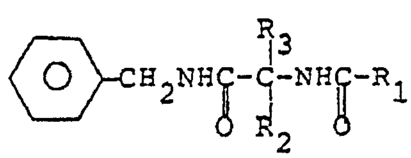

I

wherein $R_1$ is $C_1$-$C_6$ alkyl, $R_2$ and $R_3$, independently are hydrogen, $C_1$-$C_6$ alkyl or phenyl and the pharmaceuticaly acceptable salts thereof;

wherein the benzyl moiety, $R_1$, $R_2$ and $R_3$ may be substituted by halo, nitro, carboxyl, carboalkoxyl, carboxamide, cyano or thiol, alkylthio, alkoxy, alkyl, amino or phenoxy;

with the proviso that:

when $R_1$ is methyl, $R_2$ is hydrogen, and $R_3$ is hydrogen, methyl, isopropyl or isobutyl then the benzyl moiety cannot be unsubstituted benzyl; or

when $R_1$ is methyl and $R_2$ and $R_3$ are hydrogen, then the benzyl moiety cannot be substituted with 3-methoxy.

The present invention contemplates employing the above compounds in compositions of pharmaceutically acceptable dosage forms. Where the appropriate substituents are employed, the present invention also includes pharmaceutically acceptable addition salts. Moreover, the administration of an effective amount of the present compounds, in their pharmaceutically acceptable forms or the addition salts thereof, can provide an excellent regime for the treament of epilepsy, nervous anxiety, psychosis, insomnia and other related central nervous system disorders.

In accordance with the present invention, anticonvulsant compounds are provided having the general formula I. These compounds can be incorporated into pharmaceutical compositions and employed for the treatment of epilepsy and related central nervous system disorders such as anxiety, psychosis and insomnia.

The alkyl groups exemplary of the substituents are lower alkyl containing from 1 to 6 carbon atoms and may be straight chain or branched. These groups include methyl, ethyl, propyl, isopropyl, butyl, isobutyl, tertiary butyl, amyl and hexyl.

Preferred compounds of formula (I) are those wherein the benzyl moiety is substituted, $R_1$ is methyl, $R_2$ is hydrogen and $R_3$ is methyl.

Especially preferred compounds are: N-acetylphenylglycine-N'-benzylamide, N-trimethylacetyl-alanine-N'-benzylamide, N-acetyl-alanine-N'-3-fluoro-benzylamide, N-acetyl-alanine-N'-3-methoxy-benzylamide.

A further compound within the scope of the invention is N-acetyl-methionine-N'-benzylamide.

The compounds of the present invention may contain one (1) or more asymmetric carbon atoms and may exist in racemic and optically active forms. Depending upon the substituents, the present compounds may form addition salts as well. All of these forms are contemplated to be within the scope of this invention.

The compounds of the present invention can be prepared by art-recognized procedures from known compounds or readily preparable intermediates. For instance, compounds of Formula I can be prepared by reacting amines of Formula II with an acylating derivative of a carboxylic acid of Formula III under amide forming conditions:

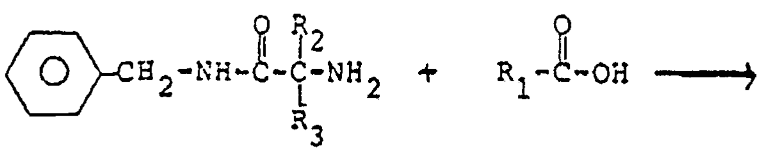

I

II III

wherein $R_1$, $R_2$, $R_3$ are as defined hereinabove.

The amide forming conditions referred to herein involve the use of known derivatives of the described acids, such as the acylhalides, (e.g.,

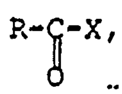

wherein X is Cl, Br) anhydrides (e.g.,

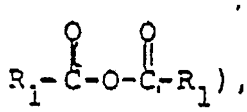

mixed anhydrides, lower alkyl esters, carbodiimides and carbonyldimidazoles. It is preferred that the acylating derivative used is the anhydride,

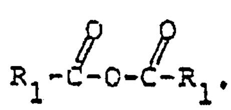

As in any organic reaction, solvents can be employed such as methanol, ethanol, propanol, acetone, tetrahydrofuran, dioxane, dimethylformamide, dichloromethane and chloroform. The reaction is normally effected at or near room temperature, although temparatures from 0 °C up to the reflux temperature of the reaction mixture can be employed.

As a further convenience, the amide forming reaction can be effected in the presence of a base, such as tertiary organic amine, e.g., trimethylamine, pyridine and picolines, particularly where hydrogen halide is formed by the amide forming reaction, e.g., aryl halide and the amine of Formula II. Of course, in those reactions where hydrogen halide is produced, any of the commonly used hydrogen halide acceptors can also be used.

The various substituents on the present new compounds, e.g., as defined in $R_1$, $R_2$ and $R_3$ can be present in the starting compounds, added to any one of the intermediates or added after formation of the final products by the known methods of substitution or conversion reactions. For example, the nitro groups can be added to the aromatic ring by nitration and the nitro group converted to other groups, such as amino by reduction, and halo by diazotization of the amino group and replacement of the diazo group. Alkanoyl groups can be substituted onto the aryl groups by Friedel-Crafts acylation. The acyl groups can be then transformed to the corresponding alkyl groups by various methods, includeing the Wolff-Kishner reduction and Clemmenson reduction. Amino groups can be alkylated to form mono and dialkylamino groups; and mercapto and hydroxy groups can be alkylated to form corresponding ethers. Primary alcohols can be oxidized by oxidizing agents known in the art to form carboxylic acids or aldehydes, and secondary alcohols can be oxidized to form ketones. Thus, substitution or alteration reactions can be employed to provide a variety of substituents throughout the molecule of the starting material, intermediates, or the final product.

In the above reactions, if the substituents themselves are reactive, then the substituents can themselves be protected according to the techniques known in the art. A variety of protecting groups known in the art may be employed. Examples of many of these possible groups may be found in "Protective Groups in Organic Synthesis," by T.W. Green, John Wiley & Sons, 1981.

The present compounds obviously exist in stereoisomeric forms and the procucts obtained thus can be mixtures of the isomers, which can be resolved. Alternatively, by selection of specific isomers as starting compounds, the preferred stereoisomer can be produced.

The active ingredients of the therapeutic compositions and the compounds of the present invention exhibit excellent anticonvulsant activity when administered in amounts ranging from 10 mg to 100 mg per kilogram of body weight per day. A preferred dosage regimen for optimum results would be from 20 mg to 50 mg per kilogram of body weight per day, and such dosage units are employed that a total of from 1.0 gram to 3.0 grams of the active compound for a subject of 70 kg of body weight are administered in a 24-hour period. This dosage regimen may be adjusted to provide the optimum therapeutic response and is preferably administered three times a day in dosages of 600 mg per administration. For example, several divided doses may be administered daily or the dose may be proportionally reduced as indicated by the exigencies of the therapeutic situation. A decided practical advantage is that the active compound may be administered in an convenient manner such as by the oral, intraveneous (where water soluble), intramuscular or subcutaneous routes.

The active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups and wafers. Such compositions and preparations should contain at least 1% of active compound. The percentage of the compositions and preparations may, of course, be varied and may conveniently be between about 5 to 80 % of the weight of the unit. The amount of active compound in such therapeutically useful compositions is such that a suitable dosage will be obtained. Preferred compositions or preparations according to the present invention are prepared so that an oral dosage unit form contains between 5 and 1000 mg of active compound.

The tablets, troches, pills and capsules may also contain the following: A binder such as gum tragacanth, acacia, corn starch or gelatin; excipients such as dicalcium phosphate; a disintegrating agent such as corn starch, potato starch and alginic acid; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a

sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimerosal.In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 5 to 1000 mg, with from 250 to 750 mg being preferred. Expressed in proportions, the active compound is generally present in from 10 to 750 mg/ml of carrier. In the case of compositions containing supplementary active ingredients, the dosages are determined by reference to the usual dose and manner of administration of the said ingredients.

For a better understanding of the present invention together with other and further objects, reference is made to the following description and example.

EXAMPLE I

General Methods. Melting points were determined with a Thomas-Hoover melting point apparatus and

are uncorrected. Infrared spectra (IR) were run on a Beckman IR-4250 spectrophotometer and calibrated against the l601-$cm^{-1}$ band of polystyrene. Absorption values are expressed in wave numbers ($cm^{-1}$). Proton nuclear magnetic resonance ($^1H$ NMR) spectra were recorded on Varian Associates Models T-60 and FT-80A NMR spectrometers. Carbon nuclear magnetic resonance ($^{13}C$ NMR) spectra were run on a Varian associates Models FT-80A instrument. Chemical shifts are in parts per million (δ values) relative to $Me_4Si$, and coupling constants (J values) are in hertz. Mass spectral data were obtained at an ionizing voltage of 70 eV on a Hewlett-Packard 5930 gas chromatograph-mass spectrometer. High-resolution (EI mode) mass spectra were performed by Dr. James Hudson at the Department of Chemistry, University of Texas at Austin, on a CEC21-110B double-focusing magnetic-sector spectrometer at 70 eV. Elemental analyses were obtained at Spang Microanalytical Laboratories, Eagle Harbor, MI.

The solvents and reactants were of the best commercial grade available and were used without further purification unless noted. All anhydrous reactions were run under nitrogen, and all glassware was dried before use.

Preparation of N-Acetyl-D,L-alanine-N'-benzylamide.

Acetic anhydride (2.20 g, 0.022 mol) was slowly added to a methylene chloride solution (30 mL) of D,L-alanine-N-benzylamide (3.80 g, 0.021 mol) and allowed to stir at room temperature (3 h). The mixture was then successively washed with $H_2O$ (15 mL), 1% aqueous NaOH (15 mL) and $H_2O$ (15 mL), dried ($Na_2SO_4$) and concentrated in vacuo. The residue was recrystallized from $CH_2Cl_2$.

Yield: 2.50 g (54%).

mp 139-241° C.

$^1H$ NMR (DMSO-$d_6$): δ 1.22 (d,J = 7.1 Hz, 3H), 1.84 (s, 3H), 4.04-4.50 (m, 3H), 7.26 (s, 5H), 8.11 (br d,J = 7.3 Hz, 1H), 8.42 (br t,J = 6 Hz, 1H).

$^{13}C$ NMR (DMSO-$d_6$): 18.2, 22.4, 41.9, 48.2, 126.5, 126.9, 128.1, 139.4, 168.9, 172.4 ppm.

IR ($CHCl_3$) 3440, 3300, 3005, 1660, 1515 $cm^{-1}$.

Mass spectrum (Cl mode), n/e: 221 (P+1); mol wt 220.1208

Preparation of N-Acetylglycine-N'-benzylamide.

The D,L-amino acid amide (11 mmol) was dissolved in dichloromethane (15mL) and then acetic anhydride (1.23 g, 1.40 mL, 12 mmol) was added dropwise. The solution was stirred at room temperature (4-6 h) and then concentrated to dryness. The residue was recrystallized from chloroform/ hexane.

Yield: 1,84 9 (81%).

mp 140-142° C.

$^1H$ NMR (DMSO-$d_6$): δ 1.88 (s, 3H), 3.74 (d,J = 5.3 Hz, 2H), 4.30 (d,J = 5.1 Hz, 2H) , 7.27 (s, 5H) , 8.37 (s, 1H), 8.75 (S. 1H).

$^{13}c$ NMR (DMSO-$d_6$): 22.5, 42.0, 42.5, 126.6, 127.1 (2C), 128.1 (2C) ,139.3, 169.0, 169.6 ppm.

IR (KBr): 3060, 1655, 1640, 1560, 1535, 1450, 1300, 740, 710 $cm^{-1}$.

Mass spectrum, m/e (relative intensity): 147 (12), 106 (100), 91 (75), 73 (50).

Elemental analysis

| | |
|---|---|
| Calculated for $C_{11}H_{14}N_2O_2$ | 64.05% C; 6.86% H; 13.58% N. |
| Found | 64.03% C; 6.79% H; 13.61% N. |

Preparation of N-Acetyl-D,L-valine-N'-benzylamide.

The D,L-amino acid amide (11 mmol) was dissolved in dichloromethane (15mL) and then acetic anhydride (1.23 g, 1.40 mL, 12 mmol) was added dropwise. The solution was stirred at room temperature (4-6 h) and then concentrated to dryness. The residue was recrystallized from chloroform/ hexane.
Yield: 2.35 g (86%).
mp 192-193° C.
$^1$H NMR (DMSO-$d_6$): δ 0.83 (d,J = 6.7 Hz, 6H), 1.87 (s, 3H), 1.73-2.09(m, 1H), 4.11(d,J = 8.9 Hz, 1H),4.27 (d,J = 5.9 Hz, 2H), 7.26 (s, 5H), 7.89 (d,J = 8.8Hz, 1H), 8.84 (t,J = 5.8 Hz, 1H).
$^{13}$C NMR (DMSO-$d_6$) : 18.1, 19.2, 22.4, 30.2, 41.9, 57.8, 126.6, 127.1 (2C), 128.1 (2C) 139.4, 169.2, 171.1 ppm.
IR (KBr) : 1620, 1540, 1530, 1450, 1380, 1290, 745, 690 cm$^{-1}$.
Mass spectrum, m/e (relative intensity): 142 (16), 114 (43), 106 (29), 91 (57), 72 (100), 55 (29).

Elemental analysis

| | | |
|---|---|---|
| Calculated for $C_{14}H_{20}N_2O_2$ | 67.70% C; 8.13% H; 11.28% N. |
| Found | 67.58% C; 8.05% H; 11.10% N. |

Preparation of N-Acetyl-D, L-phenylglycine-N'-benzylamide.

The D,L-amino acid amide (11 mmol) was dissolved in dichloromethane (15mL) and then acetic anhydride (1.23 g, 1.40 mL, 12 mmol) was added dropwise. The solution was stirred at room temperature (4-6 h) and then concentrated to dryness. The residue was recrystallized from chloroform/ hexane.
Yield: 2.05 g (66%).
np 202-203–C.
$^1$H NMR (DMSO-$d_6$): δ 1.91 (s, 3H) , 4:27 (d,J = 5.6 Hz, 2H), 5.50 (d,J = 7.9 Hz, IH) , 7.21 (s, 5H) , 7.36 (s, 5H,) 8.38-8.86 (m, 2H).
$^{13}$C NMR (DMSO-$d_6$): 22.3, 42.0, 56.3, 126.6 (2C) , 127.0, 127.1 (2C) , 127.4 (2C) , 128.1 (2C) , 138.9, 139.0, 168.9, 169.9 ppm.
IR (KBr): 3020, 1655, 1580, 1530, 1450, 1265, 745, 690 cm$^{-1}$.
Mass spectrum, m/e (relative intensity): 283 (20) , 264 (21), 149 (100) , 131 (20) , 118 (34) , 106 (92) , 91 (70) , 79 (56), 77 (54), 65 (45), 51 (37).

Elemental analysis

| | |
|---|---|
| Calculated for $C_{17}H_{18}N_2O_2$ | 72.31% C; 6.44% H; 9.92% N. |
| Found | 72.49% C; 6.47% H; 9.89% N. |

Preparation of N-Acetyl-D,L-alanine-N'-(3-methoxy-)benzylamide

The D,L-amino acid amide (11 mmol) was dissolved in dichloromethane (15mL) and then acetic anhydride (1.23 g, 1.40 mL, 12 mmol) was added dropwise. The solution was stirred at room temperature (4-6 h) and then concentrated to dryness. The residue was recrystallized from chloroform/ hexane.
Yield: 0.47 g (17%).
mp 112-115° C.
$^1$H NMR (DMSO-$d_6$): δ 1.23 (d,J = 7. 1 Hz, 3H) , 1.85 (s, 3H,) 3.73 (s, 3H) 3.99-4.48 (m, 1H), 4.25 (d,J = 6.1 Hz, 2H) , 6.58-7.35 (m, 4H) , 8.05 (d,J = 7.4 Hz, 1H) , 8.35

(t,J = 6.0 Hz, 1H).
$^{13}C$ NMR (DMSO-$d_6$): 18.1, 22.5, 41.8, 48.3, 54.9, 112.2, 112.3, 119.0, 129.2, 141.0, 159.3, 169.0, 172.4 ppm.
IR (KBr): 3270, 3065, 1625, 1580, 1450, 1260, 1150, 1095, 900, 775, 700, 690 $cm^{-1}$.

Elemental analysis

| | | |
|---|---|---|
| Calculated for $C_{13}H_{18}N_2O_3$ | 62.37% C; 7.26% H; 11.19% N. |
| Found | 62.29% C; 7.13% H; 11.08% N. |

Preparation of N-Trimethylacetyl-D,L-alanine-N'-benzylamide.

D,L-Alanine-N-benzylamide (2.45 g, 13.75 mmol) was dissolved in dichloromethane (20 mL) and isobutyric anhydride (2.17 g, 2.28 mL, 13.75 mmol) was added dropwise. The solution was stirred at room temperature (1 h) and then heated to reflux (18 h). After cooling to room temperature, the solution was concentrated to dryness and the solid residue was recrystallized from benzene/petroleum ether (30-60° C).
Yield: 1.37 g (40%).
mp 123-124°C.
$^1H$ NMR (DMSO-$d_6$): δ 1.12 (s, 9H) , 1.27 (d,J = 7.1 Hz, 3H), 4.23-4.42 (m, 1H), 4.31 (d,J = 5.4 Hz, 2H), 7.23-7.30 (m, 5H) , 7.38 (d,J = 7.4 Hz, 1H), 8.26 (t,J = 5.5 Hz, 1H).
$^{13}C$ NMR (DMSO-$d_6$): 18.1, 27.2 (3C) , 37.9, 42.0, 48.4, 126.6, 127.0 (2C) , 128.2 (2C) , 139.4, 172.5, 177.1 ppm.
IR (KBr) : 3300, 3035, 1645, 1530, 1455, 1250, 745, 695 $cm^{-1}$.

Elemental analysis

| | |
|---|---|
| Calculated for $C_{15}H_{22}N_2O_2$ | 68.66% C; 8.47% H; 10.68% N. |
| Found | 68.91% C; 8.14% H; 10.61% N. |

Preparation of N-Acetyl-D,L-methionine-N-benzylamide.

N-Acetyl-D,L-methionine (4.78 g, 25 mmol) was combined with acetonitrile (75 mL) and the mixture was placed into an ice/salt water bath (-5° C). Triethylamine (2.53 g, 3.48 mL, 25 mmol) was added dropwise, followed by ethyl chloroformate (2.71 g, 2.39 mL, 25 mmol). All additions were done slowly so that the temperature of the mixture did not rise above 0° −C. The mixture was then stirred at -5° C (20 min). Benzylamine (3.00 g, 3.06 mL, 28 mmol) in acetonitrile (5 mL) was added dropwise and the mixture was stirred at -5° C (1 h) and then room temperature (18 h).

The mixture was filtered and a white precipitate was collected and dried in vacuo and identified as the desired product ($^1H$ NMR and $^{13}C$ NMR analyses). The filtrate was concentrated in vacuo and the residue was combined with hot tetrahydrofuran (50 mL) and cooled in the freezer (3 h), resulting in the formation of a white precipitate. The mixture was filtered and the precipitate was collected, dried in vacuo, and identified as triethylammonium hydrochloride (lit. mp5 254° C).

The latter filtrate containing tetrahydrofuran was concentrated in vacuo and the resulting residue was purified by flash column chromatography (ethyl acetate). A white solid ($R_f$ = 0.50, ethyl acetate) was isolated and was 1 identified as the desired product ($^{1H}$ NMR and $^{13}C$ NMR analyses). The two solids identified as N-acetyl-D,Lmethionine-N-benzylamide were combined and recrystallized from benzene/petroleum ether (30-60°C). Yield: 2.98 g (43%).
mp 134-135° C.
$^1H$ NMR (DMSO-$d_6$): δ 1.69-1.94 (m, 2H) , 1.87 (s, 3H) , 2.02 (s,

3H) , 2.29-2.59 (m, 2H) , 4.10-4.53 (m, 1H) , 4.29 (d,J = 6.0 Hz, 2H) , 7.26 (s, 5H) , 8.12 (d,J = 8.5 Hz, 1H) , 8.47 (t,J = 6.0 Hz, 1H).

$^{13}$C NMR DMSO-$d_6$): 14.6, 22.5, 29.7, 31.8, 42.0, 52.0, 126.6, 127.0 (2C) , 128.2 (2C) , 139.4, 169.5, 171.4 ppm.

IR (KBr): 3280, 1630, 1545, 1385, 1290, 750, 700 $cm^{-1}$.

Elemental analysis

| | | |
|---|---|---|
| Calculated for $C_{14}H_{20}N_2O_2S$ | 59.96% C; 7.20% H; 9.99% N. |
| Found | 60.02% C; 7.14% H; 9.91% N. |

Preparation of N-Acetyl-alanine-N$^1$-3-fluoro-benzylamide.

N-Acetyl-alanine (3.28 9, 25 mmol) was combined with acetonitrile (100 mL) and the mixture was placed into an ice/salt bath at -5° C. Triethylamine (2.53 g, 3.5 mL, 25 mmol) was added dropwise followed by the addition of ethyl chloroformate (2.71 g, 2.40 mL, 25 mmol). All additions were done slowly so that the temperature of the mixture did not rise above 0° C. The mixture was then stirred at -5° C for 20 minutes. 3-Fluoro-benzylamine (3.58 g, 28 mmol, 5 mL) and acetonitrile was added dropwise and was stirred at -5° C for one hour and then at room temperature for 18 hours. The reaction became homogenous during this time interval.

The solution was concentrated in vacuo and the residue was combined with hot tetrahydrofuran (100 mL) and cooled in the freezer for 3 hours resulting in the formation of a white precipitate. The mixture was filtered and the precipitate was collected, dried in vacuo and identified as ethylamnonium hydrochloride (3.51 g, mp 253-257° C). The filtrate was concentrated in vacuo and the resulting yellow solid was recrystallized from chloroform/diethyl ether.

Yield: 3.22 g (54%).

mp 120-121° C.

$^1$H NMR (DMSO-$d_6$): δ 1.27 (d,J = 7.1 Hz, 3H), 1.90 (s, H), 4.23-4.41 (m, 1H), 4.33 (d,J = 6.1 Hz, 2H), 7.05-7.37 (m, 4H), 8.19 (d,J = 7.1 Hz, 1H), 8.53 (t,J = 6.1 Hz, 1H).

$^{13}$C NMR (DMSO-$d_6$): 17.9, 22.4, 41.5, 48.5, 113.3 (d,J = 24.4 Hz), 113.5 (d,J = 21.7 Hz), 122.8, 130.1 (d,J = 7.9 Hz), 142.4 (d,J = 7.4 Hz), 162.3 (d,J = 243.6 Hz), 169.6, 172.8.

IR (KBr): 3280, 1645, 1545, 1450, 745, 680. Mass spectrum, m/e (relative intensity) : 238 (18) , 151 (22), 124 (49) , 114 (47) , 109 (100) , 87 (76) 72 (27).

Elemental analysis

| | |
|---|---|
| Calculated | 68.40% C; 6.30% H; 11.76% N. |
| Found | 60.55% C; 6.32% H; 11.71% N. |

Pharmacology. The following compounds were tested for anticonvulsant activity using male Carworth Farms #1 mice:

N-Acetyl-D,L-alanine-N'-benzylamide
N-Aoetyl-D,L-phenylglycine-N'-methylamide
N-Acetylglycine-N-benzylamide
N-Acetyl-D,L-valine-N-benzylamide
N-Acetyl-D,L-phenylglycine-N-benzylamide
N-Acetyl-D,L-alanine-N-(3-methoxy)-benzylamide
N-Trimethylacetyl-D,L-alanine-N-benzylamide

N-Acetyl-D,L-methionine-N-benzylamide

N-Acetyl-alanine-N'-3-fluoro-benzylamide The compound was given in four dose levels (30, 100, 300 and 600 mg) and subsequently compared with phenytoin, mephenytoin and phenacemide (see Table I). Seizures were then artificially induced by either electroshock or Pentylenetetrazole. Maximal electroshock seizures (MES) are elicited with a 60 cycle alternating current of 50 mA intensity (5-7 times that necessary to elicit minimal electroschock seizures) delivered for 0.2 sec via corneal electrodes. A drop of 0.9% saline is instilled in the eye prior to application of the electrodes so as to prevent the death of the animal. Protection in this test is defined as the abolition of the hind limb tonic extension component of the seizure. The Subcutaneous Pentylenetetrazole (Metrazol$^{R}$) Seizure Threshold Test (sc Met) entailed the administration of 85 mg/kg of pentylenetetrazole as a 0.5% solution subcutaneously in the posterior midline. This amount of pentylenetetrazole is expected to produce seizures in greater than 95% of mice. The animal is observed for 30 minutes. Protection is defined as the failure to observe even a threshold seizure (a single episode of clonic spasms of at least 5 sec duration). The effects of the compounds on forced and spontaneous motor activity were evaluated in mice using the Rotorod Test (Tox). The animal is placed on a one-inch diameter knurled plastic rod rotating at 6 rpm after the administration of the drug. Normal mice can remain on a rod rotating at this speed indefinitely. Neurologic toxicity is defined as the failure of the animal to remain on the rod for one minute. The MES and sc Met Tests were conducted on single animals while four mice were utilized for the Tox Test. Table I includes an evaluation of the Median Effective Dose (ED50) and the Median Toxic Dose (TD50) of representative compounds. Mice were tested with varying doses of the anticonvulsant to define the limits of complete protection (or toxicity) and no protection (or no toxicity), as well as three points in between these limits. The Median Effective Dose (ED50) is defined as the dose which produced the desired endpoint in 50% of the animals. The Median Toxicity Dose (TD50) is the dose which elicited evidence of minimal neurological toxicity in 50% of the animals.

The animals exhibited no anticonvulsant activity at doses of 30 mg/kg or less. More significantly, the dose effect behavior of N-acetyl-D,L-alanine-N'-benzylamide, the D and L, isomers and N-acetyl-D,L-phenylglycine-N'-benzylamide was evaluated using the above-described procedures by the administration of varying dose levels, treating normally eight mice at each dose. As indicated by Table I, N-acetyl-D,L-alanine-N'-benzylamide was shown to have even more potent anticonvulsant activity than phenacemide with the same approximate level of toxicity.

TABLE I

Comparative Median Effective Dosage

| Compound | Tox TD50 mg/kg | MES ED50 mg/kg | sc Met ED50 mg/kg |
|---|---|---|---|
| N-acetyl-D,L-alanine-N'-benzyl amide | 454 (417-501)* | 77 (67-89)* | unmeasurable |
| N-acetyl-D-alanine-N'-benzylamide | 214 (148-262)* | 55 (50-60)* | 55 (43-67)* |
| N-acetyl-L-alanine-N'-benzylamide | 841 (691-594)* | 548 (463-741)* | unmeasurable |
| N-acetyl-D,L-phenylglycine-N'-benzylamide | 97 (80-118)* | 20 (17-24)* | unmeasurable |
| phenytoin | 66 | 10 | not effective |
| mephenytoin | 154 | 61 | 31 |
| phenacemide | 421 (337-549)* | 87 (74-100)* | 116 (71-150)* |

* 95% confidence intervals

## Claims

1. A compound having the general formula (I):

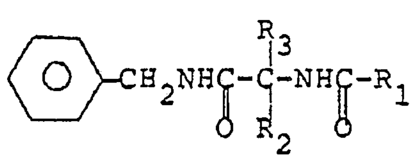

I

wherein $R_1$ is $C_1$-$c_6$ alkyl, $R_2$ and $R_3$, independently are hydrogen, $C_1$-$C_6$ alkyl or phenyl and the pharmaceuticaly acceptable salts thereof;

wherein the benzyl moiety, $R_1$, $R_2$ and $R_3$ may be substituted by halo, nitro, carboxyl, carboalkoxyl, carboxamide, cyano or thiol, alkylthio, alkoxy, alkyl, amino or phenoxy;

with the proviso that:

when $R_1$ is methyl, $R_2$, is hydrogen, and $R_3$ is hydrogen, methyl, isopropyl or isobutyl then the

benzyl moiety cannot be unsubstituted benzyl; or
when $R_1$ is methyl and $R_2$ and $R_3$ are hydrogen, then the benzyl moiety cannot be substituted with 3-methoxy.

**2.** The compound of claim 1 wherein the benzyl moiety is substituted, $R_1$ is methyl, $R_2$ is hydrogen and $R_3$ is methyl.

**3.** A compound according to any of claims 1 to 2 which is one of the following:
N-acetyl-phenylglycine-N'-benzylamide
N-trimethylacetyl-alanine-N'-benzylamide
N-acetyl-alanine-N'-3-fluoro-benzylamide
N-acetyl-alanine-N'-3-methoxy-benzylamide

**4.** N-acetyl-methionine-N'-benzylamide

**5.** The compound of claims 1 to 4 in the D isomer, L isomer or D, L racemate form.

**6.** An anticonvulsant composition comprising a compound of the formula:

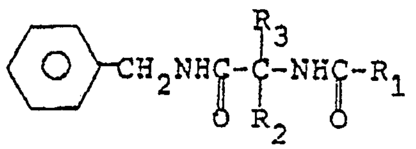
I

wherein $R_1$ is $C_1$-$C_6$ alkyl, $R_2$ and $R_3$, independently are hydrogen, $C_1$-$C_6$ alkyl or phenyl and the pharmaceutically acceptable salts thereof;
wherein the benzyl moiety, $R_1$, $R_2$ and $R_3$ may be substituted by halo, nitro, carboxyl, carboalkoxyl, carboxamide, cyano or thiol, alkylthio, alkoxy, alkyl, amino or phenoxy;

and a pharmaceutically acceptable carrier.

**7.** The composition according to Claim 6 which is one of the following:
N-acetyl-alanine-N'-benzylamide;
N-acetyl-phenylglycine-N'-benzylamide;

**8.** The use of a compound of general formula (I) according to claims 1 to 5 for the preparation of a composition for treating epilepsy, nervous anxiety, psychosis, insomnia and related nervous disorders.

Claims for the following Contracting State: Austria

**1.** A process for the preparation of a compound of formula (I)

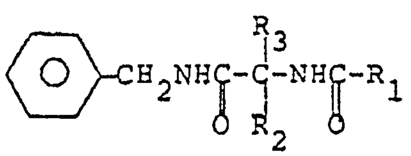
(I )

wherein $R_1$ is $C_1$-$C_6$ alkyl, $R_2$ and $R_3$, independently are hydrogen, $C_1$-$C_6$ alkyl or phenyl and the pharmaceuticaly acceptable salts thereof;
wherein the benzyl moiety, $R_1$ $R_2$ and $R_3$ may be substituted by halo, nitro, carboxyl, carboalkoxyl, carboxamide, cyano or thiol, alkylthio, alkoxy, alkyl, amino or phenoxy;
with the proviso that:

when $R_1$ is methyl, $R_2$ is hydrogen, and $R_3$ is hydrogen, methyl, isopropyl or isobutyl then the benzyl moiety cannot be unsubstituted benzyl; or
when $R_1$ is methyl and $R_2$ and $R_3$ are hydrogen, then the benzyl moiety cannot be substituted with 3-methoxy,
which comprises reacting an amine of formula (II)

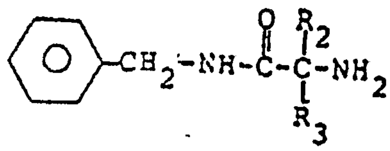 (II)

in which $R_2$ and $R_3$ have the above meaning with an acylating derivative of formulae

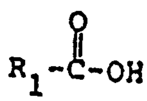 , 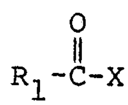 ,

wherein X is Cl or Br or

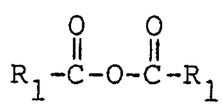

in which $R_1$ has the above meaning,
under amide forming conditions and optionally removing protecting groups when present; and optionally introducing substituents into the compounds by substitution or conversion reactions and optionally forming pharmaceutically acceptable salts of said compounds.

2. A process according to claim 1 wherein the benzyl moiety is substituted, $R_1$ is methyl, $R_2$ is hydrogen and $R_3$ is methyl.

3. A process according to claim 1 for the production of:
N-acetyl-phenylglycine-N'-benzylamide
N-trimethylacetyl-alanine-N'-benzylamide
N-acetyl-alanine-N'-3-fluoro-benzylamide
N-acetyl-alanine-N'-3-methoxy-benzylamide.

4. A process according to claim 1 for the production of:
N-acetyl-methionine-N'-benzylamide.

5. A process according to claim 1 for the production of a compound in the D isomer, L isomer or D,L racemate form.

6. Use of a compound of general formula (I) according to claim 1 for the preparation of a composition for treating epilepsy, nervous anxiety, psychosis, insomnia and related nervous disorders.

## Revendications

1. Composé ayant la formule générale (I) :

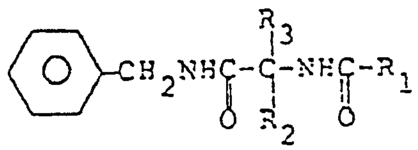

I

dans laquelle :

- R1 représente alkyle en $C_1$-$C_6$,
- $R_2$ et $R_3$ représentent indépendamment hydrogène, alkyle en $C_1$-$C_6$ ou phényle, et ses sels pharmaceutiquement acceptables ; la fraction benzyle, $R_1$, $R_2$ et $R_3$ pouvant être substitués par halo, nitro, carboxyle, carboalcoxyle, carboxamide, cyano ou thiol, alkylthio, alcoxy, alkyle, amino ou phénoxy; avec la condition que :

lorsque $R_1$ représente méthyle, $R_2$ représente hydrogène, et $R_3$ représente hydrogène, méthyle, isopropyle ou isobutyle, la fraction benzyle ne peut pas représenter benzyle non-substitué ; ou lorsque $R_1$ représente méthyle et $R_2$ et $R_3$ représentent hydrogène, la fraction benzyle ne peut pas être substituée par méthoxy en position 3.

2. Composé selon la revendication 1, dans lequel la fraction benzyle est substituée, $R_1$ représente méthyle, $R_2$ représente hydrogène, et $R_3$ représente méthyle.

3. Composé selon l'une des revendications 1 et 2, qui est l'un des suivants :
   ± le N-acétyl-phénylglycine-N'-benzylamide
   ± le N-triméthylacétyl-alanine-N'-benzylamide
   ± le N-acétyl-alanine-N'-fluoro-3 benzylamide
   ± le N-acétyl-alanine-N'-méthoxy-3 benzylamide.

4. Le N-acétyl-méthionine-N'-benzylamide.

5. Composé selon l'une des revendications 1 à 4 sous la forme de l'isomère D, de l'isomère L, ou du racémate D, L.

6. Composition anticonvulsivante comprenant un composé de la formule :

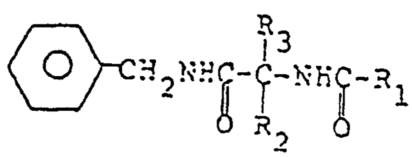

I

dans laquelle :

- $R_1$ représente alkyle en $C_1$-$C_6$,
- $R_2$ et $R_3$ représentent indépendamment hydrogène, alkyle en $C_1$-$C_6$ ou phényle ; et ses sels pharmaceutiquement acceptables ; la fraction benzyle, $R_1$, $R_2$ et $R_3$ pouvant être substitués par halo, nitro, carboxyle, carboalcoxyle, carboxamide, cyano ou thiol, alkylthio, alcoxy, alkyle, amino ou phénoxy; et un support pharmaceutiquement acceptable.

7. Composition selon la revendication 6, qui comprend l'un des composés suivants :
   ± le N-acétyl-alanine-N'-benzylamide ;
   ± le N-acétyl-phénylglycine-N'-benzylamide.

8. Utilisation d'un composé de la formule générale (I) selon l'une des revendications 1 à 5, pour la préparation d'une composition pour le traitement de l'épilepsie, de l'anxiété nerveuse, de la psychose, de l'insomnie et des troubles nerveux apparentés.

Revendications pour l'Etat contractant suivant: Autriche

**1.** Procédé de préparation d'un composé de formule (I) :

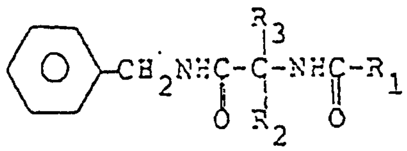

(I)

dans laquelle :

- $R_1$ représente alkyle en $C_1$-$C_6$,
- $R_2$ et $R_3$ représentent indépendamment hydrogène, alkyle en $C_1$-$C_6$ ou phényle,
  et ses sels pharmaceutiquement acceptables ; la fraction benzyle, $R_1$, $R_2$ et $R_3$ pouvant être substitués par halo, nitro, carboxyle, carboalcoxyle, carboxamide, cyano ou thiol, alkylthio, alcoxy, alkyle, amino ou phénoxy;
  avec la condition que :
- lorsque $R_1$ représente méthyle, $R_2$ représente hydrogène, et $R_3$ représente hydrogène, méthyle, isopropyle ou isobutyle, la fraction benzyle ne peut pas représenter benzyle non-substitué ; ou
- lorsque $R_1$ représente méthyle et $R_2$ et $R_3$ représentent hydrogène, la fraction benzyle ne peut pas être substituée par méthoxy en position 3, qui comprend la réaction d'une amine de formule (II)

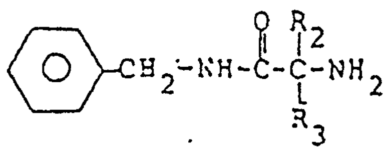

(II)

dans laquelle $R_2$ et $R_3$ ont la signification ci-dessus avec un dérivé acylant des formules :

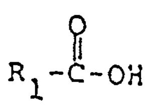 , 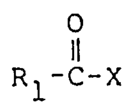 ,

où X représente Cl ou Br ou

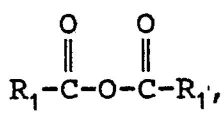

où $R_1$ a la signification ci-dessus,
dans des conditions de formation d'un amide, et, facultativement, l'élimination des groupes protecteurs s'ils sont présents ; et, facultativement, l'introduction de substituants dans les composés par des réactions de substitution ou de conversion et, facultativement, la formation de sels pharmaceutiquement acceptables desdits composés.

**2.** Procédé selon la revendication 1, dans lequel la fraction benzyle est substituée, $R_1$ représente méthyle, $R_2$ représente hydrogène, et $R_3$ représente méthyle.

**3.** Procédé selon la revendication 1 pour la fabrication de :

± le N-acétyl-phénylglycine-N'-benzylamide
± le N-triméthylacétyl-alanine-N'-benzylamide

± le N-acétyl-alanine-N'-fluoro-3 benzylamide
± le N-acétyl-alanine-N'-méthoxy-3 benzylamide.

4. Procédé selon la revendication 1 pour la production de N-acétyl-méthionine-N'-benzylamide.

5. Procédé selon la revendication 1 pour la production d'un composé sous la forme d'isomère D, d'isomère L, ou de racémate D, L.

6. Utilisation d'un composé de formule générale (I) selon la revendication 1, pour la préparation d'une composition pour le traitement de l'épilepsie, de l'anxiété nerveuse, de la psychose, de l'insomnie et des troubles nerveux apparentés.

## Ansprüche

1. Verbindung mit der allgemeinen Formel (I):

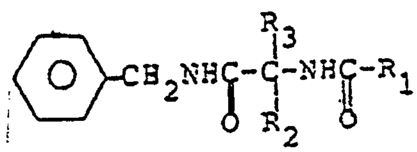

I

wobei $R_1$ $C_1 = C_6$ Alkyl, $R_2$ und $R_3$ unabhängig voneinander Wasserstoff, $C_1$-$C_6$ Alkyl oder Phenyl darstellen, und die pharmazeutisch verträglichen Salze davon;
wobei der Benzylteil, $R_1$, $R_2$ und $R_3$ durch Halogen, Nitro, Carboxyl, Carboalkoxyl, Carboxamid, Cyano oder Thiol, Alkylthio, Alkoxy, Alkyl, Amino oder Phenoxy substituiert sein können; unter der Voraussetzung, daß:
wenn $R_1$ Methyl ist, $R_2$ Wasserstoff ist, und wenn $R_3$ Wasserstoff, Methyl, Isopropyl oder Isobutyl ist, dann der Benzylteil kein unsubstituiertes Benzyl sein kann; oder
wenn $R_1$ Methyl ist und $R_2$ und $R_3$ Wasserstoff darstellen, dann der Benzylteil nicht durch 3-methoxy substituiert sein kann.

2. Verbindung nach Anspruch 1, wobei der Benzylteil substituiert ist, $R_1$ Methyl, $R_2$ Wasserstoff und $R_3$ Methyl sind.

3. Verbindung nach einem der Ansprüche 1 bis 2, die eine der folgenden Verbindungen darstellt:
N-acetyl-phenylglycin-N'-benzylamid
N-trimethylacetyl-alanin-N'-benzylamid
N-acetyl-alanin-N'-3-fluor-benzylamid
N-acetyl-alanin-N'-3-methoxy-benzylamid

4. N-acetyl-methionin-N'-benzylamid.

5. Verbindung nach Anspruch 1 bis 4, als D-isomer, L-Isomer oder D, L Racemat.

6. Antikonvulsive Zusammensetzung, die eine Verbindung der Formel (I):

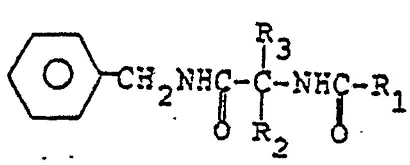

I

wobei $R^1$ $C_1$-$C_6$ Alkyl, $R_2$ und $R_3$ unabhängig voneinander Wasserstoff, $C_1$-$C_6$ Alkyl oder Phenyl sind, und die pharmazeutisch verträglichen Salze davon; wobei der Benzylteil, $R_1$, $R_2$ und $R_3$ durch Halogen, Nitro, Carboxyl, Carboalkoxyl, Carboxamid, Cyano oder Thiol, Alkylthio, Alkoxy, Alkyl, Amino oder

Phenoxy substituiert sein können; und einen pharmazeutisch verträglichen Träger umfaßt.

7. Zusammensetzung nach Anspruch 6, die eine der folgenden Verbindungen enthält:
N-acetyl-alanin-N'-benzylamid;
N-acetyl-phenylglycin-N'-benzylamid.

8. Verwendung einer Verbindung der allgemeinen Formel (I) gemäß den Ansprüchen 1 bis 5 zur Herstellung einer Zusammensetzung zur Behandlung von Epilepsie, nervöser Ängstlichkeit, Psychosen, Schlaflosigkeit und verwandten nervösen Störungen.

Patentansprüche für folgenden Vertragsstaat: AT

1. Verfahren zur Herstellung einer Verbindung mit der allgemeinen Formel (I):

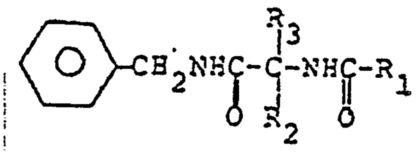

(I)

wobei $R_1$ $C_1$-$C_6$ Alkyl, $R_2$ und $R_3$ unabhängig voneinander Wasserstoff, $C_1$-$C_6$ Alkyl oder Phenyl darstellen, und den pharmazeutisch verträglichen Salze davon;
wobei der Benzylteil, $R_1$, $R_2$ und $R_3$ durch Halogen, Nitro, Carboxyl, Carboalkoxyl, Carboxamid, Cyano oder Thiol, Alkylthio, Alkoxy, Alkyl, Amino oder Phenoxy substituiert sein können;

unter der Voraussetzung, daß: wenn $R_1$ Methyl ist, $R_2$ Wasserstoff ist, und wenn $R_3$ Wasserstoff, Methyl, Isopropyl oder Isobutyl ist, dann der Benzylteil kein unsubstituiertes Benzyl sein kann; oder wenn $R_1$ Methyl ist und $R_2$ und $R_3$ Wasserstoff darstellen, dann der Benzylteil nicht durch 3-methoxy substituiert sein kann;
das die Reaktion eines Amins der Formel (II)

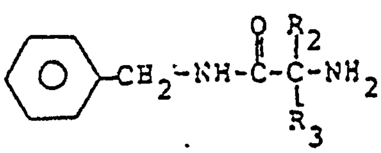

(II)

wobei $R_2$ und $R_3$ die obigen Bedeutungen besitzen, mit einem Acylierungsderivat der Formeln

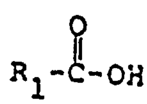 , 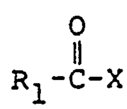 ,

wobei X Cl oder Br oder

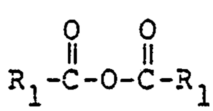

,wobei $R_1$ die obige Bedeutung hat, ist,
unter Amidbildungsbedingungen und gegebenenfalls das Entfernen vorhandener Schutzgruppen und gegebenenfalls die Einführung von Substituenten in die Verbindungen durch Substitution oder Umwandlungsreaktionen und gegebenenfalls die Bildung pharmazeutisch verträglicher Salze der Verbindungen umfaßt.

2. Verfahren nach Anspruch 1, wobei der Benzylteil substituiert ist, $R_1$ Methyl, $R_2$ Wasserstoff und $R_3$ Methyl ist.

3. Verfahren nach Anspruch 1 zur Herstellung von: N-acetyl-phenylglycin-N'-benzylamid N-trimethylacetyl-alanin-N'-benzylamid N-acetyl-alanin-N'-3-fluoro-benzylamid N-acetyl-alanin-N'-3-methoxy-benzylamid.

4. Verfahren nach Anspruch 1 zur Herstellung von: N-acetyl-methionin-N'-benzylamid.

5. Verfahren nach Anspruch 1 zur Herstellung einer Verbindung in der Form des D-Isomers, L-Isomers oder des D, L Racemats.

6. Verwendung einer Verbindung der allgemeinen Formel (I) gemäß Anspruch 1 zur Herstellung einer Zusammensetzung zur Behandlung von Epilepsie, nervöser Ängstlichkeit, Psychosen, Schlaflosigkeit und verwandten nervösen Störungen.